Europäisches Patentamt

(19) European Patent Office

Office européen des brevets

(11) Veröffentlichungsnummer: **0 196 088**
**B1**

## EUROPÄISCHE PATENTSCHRIFT

(45) Veröffentlichungstag der Patentschrift:
01.06.88

(21) Anmeldenummer: 86104161.4

(22) Anmeldetag: 26.03.86

(51) Int. Cl.⁴: **C 07 C 17/28,** C 07 C 19/02,
C 07 F 7/08, C 07 C 67/293

(54) Verfahren zur Herstellung von Verbindungen mit mehrfach durch Chlor substituiertem Alkylrest.

(30) Priorität: 27.03.85 DE 3511153

(43) Veröffentlichungstag der Anmeldung:
01.10.86 Patentblatt 86/40

(45) Bekanntmachung des Hinweises auf die Patenterteilung:
01.06.88 Patentblatt 88/22

(84) Benannte Vertragsstaaten:
AT BE CH DE FR GB IT LI NL SE

(56) Entgegenhaltungen:
EP - A - 0 131 561
DD - A - 143 424
US - A - 4 243 607

PATENT ABSTRACTS OF JAPAN, unexamined applications, Sektion C, Band 1, Nr. 53, 23. Mai 1977 THE PATENT OFFICE JAPANESE GOVERNMENT Seite 317 C 77
CHEMICAL ABSTRACTS, Band 75, Nr. 7, 16. August 1971, Columbus, Ohio, USA T. ASAHARA et al. "Telemerization of ethylene and carbon tetrachloride" Seite 303, Zusammenfassung-Nr. 48 334n

(73) Patentinhaber: WACKER-CHEMIE GMBH,
Prinzregentenstrasse 22, D-8000 München 22 (DE)

(72) Erfinder: Blum, Klaus, Dr. Dipl.-Chem., Amatiweg 5,
D-8263 Burghausen (DE)
Erfinder: Strasser, Rudolf, Dr. Dipl.-Chem., Lindacher Strasse 58, D-8263 Burghausen (DE)
Erfinder: Vogl, Günther, Burghauser Strasse 30,
D-8262 Altötting (DE)
Erfinder: Hierzegger, Roman, Regerstrasse 10,
D-8263 Burghausen (DE)

BAD ORIGINAL

## Beschreibung

Die Erfindung betrifft ein verbessertes Verfahren zur Herstellung von Verbindungen mit mehrfach durch Chlor substituiertem Alkylrest, insbesondere von Tetrachloralkanen und Tetrachloralkylverbindungen durch Addition von Tetrachlormethan an ethylenisch ungesättigte Verbindungen.

Solche stets in Gegenwart von Katalysatoren durchgeführten Verfahren zur Addition von Tetrachlormethan an ethylenische Doppelbindungen sind bereits vielfach vorgeschlagen worden, jedoch waren ihre Ergebnisse bisher noch nicht zufriedenstellend, da Umsatz und/oder Selektivität zu wünschen übrigliessen oder diese Verfahren andere Nachteile aufwiesen.

Beispielsweise stellen Verfahren unter Einsatz von Katalysatoren auf der Basis radikalisch zerfallender Peroxide hohe Anforderungen an die Sicherheitsausrüstung der Reaktoren, da mit plötzlichen Druckanstiegen zu rechnen ist. Ausserdem werden in erheblichem Masse unerwünschte Nebenprodukte gebildet.

Es wurde daher bereits vielfach versucht, diese Katalysatoren durch andere Systeme zu ersetzen. Beispielsweise wurden dafür metallorganische Verbindungen von Edelmetallen wie Ruthenium vorgeschlagen, die jedoch sehr teuer sind und eine aufwendige Produktionstechnik erfordern, da Luftsauerstoff und Feuchtigkeit den Katalysator irreversibel schädigen.

Eisen (II)-, Eisen (III)-, Kupfer (I)- und Kupfer (II)-Verbindungen fördern bei Drücken über 20 bar und Temperaturen über 80° C die Addition von Tetrachlormethan an C=C-Doppelbindungen. Hierbei lässt jedoch die Selektivität zu wünschen übrig, da sich stets auch Oligomere der ethylenischen Verbindungen sowie teerartige Substanzen bilden.

Diese Katalysatorsysteme sind in der Folge durch Zusatz von Cokatalysatoren und bestimmten Lösungsmitteln modifiziert worden, um die Selektivität zu erhöhen. Zum Beispiel schlägt die US-A-4243607 ein Verfahren zur Herstellung von Tetrachloralkanen vor, das durch eine Kombination von Alkylphosphit, Eisenchlorid und einem Nitril katalysiert wird. Auch dieses Verfahren befriedigt noch nicht vollständig, da stets zumindest noch Oligomerisierung der ethylenischen Reaktionskomponente in merklichem Ausmass auftritt.

Es bestand daher die Aufgabe, ein demgegenüber verbessertes Verfahren zur Durchführung der Additionsreaktion von $CCl_4$ an C=C-Doppelbindungen zu entwickeln, die zudem nicht nur auf die Herstellung von Tetrachloralkanen beschränkt ist, sondern auch die Addition von $CCl_4$ an Vinylverbindungen mit funktionellen Gruppen bei guten Ausbeuten und guter Selektivität, vorzugsweise über 90%, gestattet.

Gegenstand der Erfindung ist ein Verfahren zur Herstellung von Verbindungen von mehrfach mit Chlor substituiertem Alkylrest, insbesondere mit 1,1,1,3-Tetrachloralkylrest, durch Addition von Tetrachlormethan an ethylenisch ungesättigte Verbindungen in Gegenwart eines Katalysatorsystems, basierend auf Metall(verbindung) und phosphororganischer Verbindung bei erhöhter Temperatur und gegebenenfalls erhöhtem Druck. Das Verfahren ist dadurch gekennzeichnet, dass die Reaktion bei Temperaturen von 50 bis 150° C, vorzugsweise 75 bis 120° C und Drücken von weniger als 20 bar (absolut), vorzugsweise 1 bis 8 bar (absolut), in Gegenwart von Eisenverbindung oder vorzugsweise metallischem Eisen, der Phosphorverbindung und von Kohlendioxid durchgeführt wird.

Durch das erfindungsgemässe Verfahren gelingt es, die Bildung von teerartigen Produkten und im allgemeinen auch die Bildung von Oligomerisierungsprodukten und anderen Nebenprodukten völlig zu unterbinden. Ausserdem kann auf den Einsatz von Lösungsmitteln und weiteren Zusätzen wie z.B. Nitrilen völlig verzichtet werden. Obgleich Eisenspuren bekanntermassen besonders aktive Dehydrohalogenierungskatalysatoren darstellen, wurden derartige Nebenreaktionen überraschenderweise bei dem erfindungsgemässen Verfahren nicht gefunden.

Als geeignete Eisenkomponente des Katalysatorsystems sind Eisen (II)- und Eisen (III)-Verbindungen zu nennen, insbesondere die Salze oder Kombinationen solcher Salze. Namentlich seien hier als einzelne Beispiele genannt: wasserfreies Eisen (II)- oder Eisen (III)-chlorid, Eisen (II)- oder Eisen (III)-nitrat, Eisen (II)-sulfat, Eisen (II)-Acetylacetonat sowie gegebenenfalls Hydrate solcher Salze, z.B. Eisenalaun, Eisen (III)chlorid-Hexahydrat, Eisen (III)citrat-Tetrahydrat, sowie Ferrocen. Bevorzugt wird jedoch statt dessen metallisches Eisen eingesetzt. Es kann in beliebiger Form verwendet werden, z.B. als Granulat, als Späne, als Ringe, als Rohre, als Kugeln, als Pulver u.ä. Da dabei die Reaktion besonders gleichmässig und schonend verläuft und die Eisenkomponente des Katalysatorsystems besonders einfach vom Produkt abgetrennt und wiederholt eingesetzt werden kann, ohne dass die Aktivität stark abfällt, haben sich die relativ kompakten Formen wie Granulat, Ringe, Rohre und Kugeln als besonders vorteilhaft herausgestellt. Die sonst bei der Katalysatorherstellung allgemein übliche Herstellung besonders oberflächenreicher Formen hat sich überraschenderweise als überflüssig und teils sogar als nachteilig erwiesen.

Die optimale Eisenmenge kann in wenigen Versuchen bestimmt werden, sie liegt beim Einsatz metallischen Eisens bevorzugt im Bereich von 1 bis 10 mmol Eisen, bezogen auf eingesetzte ethylenisch ungesättigte Verbindung. Manchmal, z.B. bei der Addition an Vinylester, hat es sich gezeigt, dass durch die Zugabe geringerer Mengen von Eisensalzen, vorzugsweise 0,1 bis 10 Mol%, bezogen auf das metallische Eisen, die Induktionsperiode verkürzt werden kann.

Als Phosphorverbindung können phosphororganische Verbindungen eingesetzt werden, in denen der Phosphor nicht in seiner höchsten Oxidationsstufe vorliegt, z.B. Phosphane und insbesondere Ester der phosphorigen Säure sowie Umsetzungsprodukte von Phosphortrichlorid mit

mehrwertigen Alkoholen wie Glykol. Besonders vorteilhaft haben sich Triethylphosphit und insbesondere Tri(2-chlorethyl)phosphit (T2-CEP) erwiesen, da hiermit die Bildung von Nebenprodukten praktisch völlig vermieden werden kann.

Die Phosphorverbindung wird vorzugsweise in Mengen von 0,1 bis 5 mmol pro Mol ethylenisch ungesättigter Verbindung eingesetzt. Insbesondere haben sich Mengen unter 4 mmol bewährt. Ein typischer Wert für die optimale Menge der Phosphorverbindung bei der Addition von $CCl_4$ an Ethylen als ethylenisch ungesättigte Verbindung liegt bei 0,4 bis 0,6 mmol pro Mol Ethylen.

Das erfindungsgemässe Verfahren wird in Gegenwart von Kohlendioxid als dritter Katalysatorkomponente durchgeführt. Es kann gasförmig oder als Trockeneis, also fest, zugegeben werden. Seine Menge (als Gas) kann vorzugsweise im Bereich von 0,1 bis 10 Vol-%, bezogen auf das Tetrachlormethan-Volumen, liegen, insbesondere bei 0,5 bis 2 Vol-%. Da das erfindungsgemässe Verfahren bevorzugt diskontinuierlich durchgeführt wird, wird das Kohlendioxid vorzugsweise vor oder bei Reaktionsbeginn in seiner gesamten Menge zugegeben, so dass der Reaktor dann verschlossen werden kann. Es ist aber natürlich möglich, die ethylenisch ungesättigte Verbindung, insbesondere sofern sie gasförmig ist, auch noch nachzudosieren, z.B. wie schon beschrieben, einen bestimmten Druck dieser Verbindung im Reaktionsgefäss aufzubauen. In diesem Fall ist es selbstverständlich nicht ausgeschlossen, ebenfalls weiteres gasförmiges $CO_2$ zuzuführen, obgleich dies üblicherweise nicht gemacht wird und daher auch nicht bevorzugt ist.

Das Tetrachlormethan wird vorzugsweise vorgelegt, wobei es möglich ist, diese Verbindung im Überschuss einzusetzen, so dass sie gleichzeitig als Lösungsmittel für die anderen Reaktionsteilnehmer dienen kann. Vorzugsweise werden das Tetrachlormethan und die ethylenisch ungesättigte Verbindung jedoch in annähernd stöchiometrischen oder insbesondere stöchiometrischen Mengen eingesetzt.

Das erfindungsgemässe Verfahren eignet sich zur Durchführung der Addition von Tetrachlormethan an Olefine wie Ethylen, Propen, 1-Buten, 2-Buten, 1-Hexen und 2-Hexen, an Vinylsilane wie Trimethoxyvinylsilan, Trichlorvinylsilan, Methyldichlorvinylsilan, Dimethyl-chlor-vinylsilan, an Vinylester gesättigter Carbonsäuren wie Vinylacetat und Vinylpropionat, an Vinylether, an Allylester gesättigter Carbonsäuren, z.B. Allylacetat, an Allylnitril, an Allylalkohol, um nur einige geeignete Beispiele zu nennen.

Das erfindungsgemässe Verfahren kann in kontinuierlich betriebenen Kaskaden oder sogar im Rohrreaktor kontinuierlich durchgeführt werden. Vorzugsweise werden jedoch diskontinuierlich betriebene Rührreaktoren, z.B. Autoklaven eingesetzt.

Die Reaktionsdauer bzw. Verweilzeit liegt üblicherweise, abhängig von der Ansatzgrösse, zwischen einer und 48 h (z.B. bei grosstechnischen Ansätzen). Typisch ist ein erreichbarer Umsatz von 90% innerhalb von 24 h.

Die optimale Ansatzzusammensetzung und die optimalen Reaktionsbedingungen, die natürlich von den eingesetzten Substanzen, insbesondere den ethylenisch ungesättigten Verbindungen abhängen, können vom Fachmann in wenigen Vorversuchen bestimmt werden, wobei generell gefunden wird, dass niedrigere Drücke wegen der Vermeidung von Nebenreaktionen günstiger sind.

Die folgenden Beispiele dienen der weiteren Erläuterung der Erfindung.

*Beispiel 1*

In einem 1-l-Autoklaven wurden 75 g Eisenringe ($\varnothing$ 3 cm, Höhe 3 cm, Wandstärke 0,8 mm), 10 ml (50 mmol) T 2-CEP, 800 ml (8,29 mol) Tetrachlormethan und 10 ml festes Kohlendioxid gefüllt. Bei Aufpressen von 10 bar Ethylen bei 120° C wurden nach 16 h Reaktionsdauer 95,3% des Tetrachlormethans zu 1,1,1,3-Tetrachlorpropan umgesetzt. Der Eisen-Bestandteil des Katalysators wurde 11mal wiederverwendet, wobei T 2-CEP und Kohlendioxid stets frisch zugegeben wurden. Beim 12. Ansatz wurden innerhalb von 16 h 82% des Tetrachlormethans zu 1,1,1,3-Tetrachlorpropan (1,1,1,3-TCP) umgesetzt.

*Beispiel 2*

Beispiel 1 wurde unter Variation des Druckes und der Temperatur wiederholt:

| Versuch Nr. | Druck (bar) | Temperatur (°C) | Reaktionsdauer (h) | Umsatz von $CCl_4$ zu 1,1,1,3-TCP (%) | Nebenprodukte (Gew%) |
|---|---|---|---|---|---|
| 2.1 | 6 | 80 | 16 | 22 | — |
| 2.2 | 6 | 90 | 16 | 55 | — |
| 2.3 | 6 | 100 | 16 | 74 | — |
| 2.4 | 6 | 120 | 16 | 94 | — |
| 2.5 | 6 | 120 | 22 | 96 | — |
| 2.6 | 12 | 120 | 22 | 92 | 4 |
| 2.7 | 15 | 120 | 22 | 90 | 8 |
| 2.8 | 20 | 120 | 22 | 89 | 10 |

## Beispiel 3

In einem 250-ml-Dreihalskolben wurden 123 g (0,8 mol) Tetrachlormethan, 96,6 g (0,8 mol) Vinyldimethylchlorsilan in Gegenwart von 3,0 g Fe-Griess und 3 ml T 2-CEP gemischt und ca. 1 l gasförmiges Kohlendioxid eingeleitet. Nach einer Reaktionsdauer von 24 h bei 80° C waren 91% der Ausgangsprodukte zu 1,1,1,3-Tetrachlorpropyldimethyl-chlorsilan umgesetzt.

Kp (0,02): 56,5-58° C, Gaschromatographische ermittelte Reinheit: 98,7%.

## Beispiel 4

In einem 1-l-Dreihalskolben wurden zu 119 g (0,80 mol) Trimethoxyvinylsilan und 123 g (0,80 mol) Tetrachlormethan 1,0 g Eisengriess, 5 ml T 2-CEP und ca. 20 ml festes Kohlendioxid gegeben. Beim Rückflusskochen stieg die Temperatur innerhalb von 8 h von 78° C auf 120° C. Es wurden nach dieser Zeit durch Destillation 139 g (0,46 mol) 1,1,1,3-Tetrachlorpropyl-trimethoxysilan erhalten.

## Beispiel 5

100 g (1 mol) Essigsäureallylester und 154 g (1 mol) Tetrachlormethan wurden mit 5 g Eisenringen, 5 ml Triethylphosphit und 10 ml festem Kohlendioxid versetzt und auf Rückfluss erhitzt. Innerhalb von 3 h stieg die Temperatur von 78° C auf 110° C. Durch Destillation im Wasserstrahlvakuum wurden 155 g 1,1,1,3-Tetrachlorbutylacetat (Kp (13): 158-161° C) erhalten.

## Patentansprüche

1. Verfahren zur Herstellung von Verbindungen mit mehrfach durch Chlor substituiertem Alkylrest durch Addition von Tetrachlormethan an ethylenisch ungesättigte Verbindungen in Gegenwart eines Katalysatorsystems, basierend auf Metall-(verbindung) und phosphororganischer Verbindung bei erhöhter Temperatur und gegebenenfalls erhöhtem Druck, dadurch gekennzeichnet, dass die Reaktion bei Temperaturen von 50 bis 150° C und Drücken von weniger als 20 bar in Gegenwart von metallischem Eisen oder Eisenverbindung, der Phosphorverbindung und von Kohlendioxid durchgeführt wird.

2. Verfahren nach Anspruch 1, dadurch gekennzeichnet, dass die Reaktion in Gegenwart von metallischem Eisen, Kohlendioxid und Triethylphosphit und/oder Tri(2-chlorethyl)phosphit durchgeführt wird.

## Claims

1. A process for preparing a compound having a polychlorinated alkyl radical by adding tetrachloromethane to an ethylenically unsaturated compound in the presence of a catalyst system based on a metal (compound) and an organophosphorous compound at elevated temperature and, if appropriate, elevated pressure, wherein the reaction is carried out at temperatures of 50 to 150° C and pressures of less than 20 bar in the presence of metallic iron or an iron compound, the phosphorous compound and carbon dioxide.

2. The process as claimed in claim 1, wherein the reaction is carried out in the presence of metallic iron, carbon dioxide and triethyl phosphite and/or tri(2-chloroethyl) phosphite.

## Revendications

1. Procédé pour préparer des composés renfermant un radical alkyle porteur de plusieurs atomes de chlore, par fixation du tétrachlorométhane sur des composés éthyléniques en présence d'un système catalytique à base d'un métal ou d'un composé de métal et d'un composé organique du phosphore, à température élevée et, éventuellement, sous pression élevée, procédé caractérisé en ce qu'on effectue la réaction à des températures de 50 à 150° C et sous des pressions inférieures à 20 bar, en présence de fer métallique ou d'un composé du fer, du composé du phosphore et de dioxyde de carbone.

2. Procédé selon la revendication 1, caractérisé en ce qu'on effectue la réaction en présence de fer métallique, de dioxyde de carbone et de phosphite de triéthyle et/ou de phosphite de tris-(chloro-2 éthyle).